# EUROPÄISCHE PATENTSCHRIFT

(11) **EP 1 294 945 B1**
(45) Veröffentlichungstag und Bekanntmachung des Hinweises auf die Patenterteilung: **30.08.2006**
(21) Anmeldenummer: 01953113.6
(22) Anmeldetag: 19.06.2001
(51) Int. Cl.: C12Q 1/68

(54) **VERFAHREN ZUM NACHWEIS VON CYTOSIN-METHYLIERUNGEN**
METHOD FOR DETECTING CYTOSINE METHYLATIONS
PROCEDE POUR LA MISE EN EVIDENCE DE METHYLATIONS DE LA CYTOSINE

(30) Priorität: 19.06.2000 DE 10029915
(43) Veröffentlichungstag der Anmeldung: 26.03.2003
(60) Teilanmeldung: 06090098.2
(73) Patentinhaber: Epigenomics AG, 10178 Berlin (DE)
(72) Erfinder: BERLIN, Kurt, 14532 Stahnsdorf (DE)
(74) Vertreter: Schubert, Klemens
(86) Internationale Anmeldenummer: PCT/DE2001/002274
(87) Internationale Veröffentlichungsnummer: WO 2001/098528

(56) Entgegenhaltungen:
- WO-A-99/28498
- US-A- 4 851 331
- PAULIN RICHARD ET AL: "Urea improves efficiency of bisulphite-mediated sequencing of 5'-methylcytosine in genomic DNA." NUCLEIC ACIDS RESEARCH, Bd. 26, Nr. 21, 1. November 1998 (1998-11-01), Seiten 5009-5010, XP002210104 ISSN: 0305-1048
- WONG DAVID J ET AL: "P16-INK4a promoter is hypermethylated at a high frequency in esophageal adenocarcinomas." CANCER RESEARCH, Bd. 57, Nr. 13, 1997, Seiten 2619-2622, XP002210105 ISSN: 0008-5472
- LANDEGREN U ET AL: "A LIGASE-MEDIATED GENE DETECTION TECHNIQUE" SCIENCE, AMERICAN ASSOCIATION FOR THE ADVANCEMENT OF SCIENCE, US, Bd. 241, Nr. 4869, 26. August 1988 (1988-08-26), Seiten 1077-1080, XP000676556 ISSN: 0036-8075
- GONZALGO M L AND JONES P A: "Rapid quantification of methylation differences at specific sites using methylation-sensitive single nucleotide primer extension (Ms-SNuPE)" NUCLEIC ACIDS RESEARCH, OXFORD UNIVERSITY PRESS, SURREY, GB, Bd. 25, Nr. 12, 1997, Seiten 2529-2531, XP002106409 ISSN: 0305-1048
- HERMAN J G ET AL: "METHYLATION-SPECIFIC PCR: A NOVEL PCR ASSAY FOR METHYLATION STATUS OF CPG ISLANDS" PROCEEDINGS OF THE NATIONAL ACADEMY OF SCIENCES OF USA, NATIONAL ACADEMY OF SCIENCE. WASHINGTON, US, Bd. 93, 1. September 1996 (1996-09-01), Seiten 9821-9826, XP002910406 ISSN: 0027-8424
- ABRAVAYA KLARA ET AL: "Detection of point mutations with a modified ligase chain reaction (Gap-LCR)." NUCLEIC ACIDS RESEARCH, Bd. 23, Nr. 4, 1995, Seiten 675-682, XP002210106 ISSN: 0305-1048
- CLARK SUSAN J ET AL: "High sensitivity mapping of methylated cytosines." NUCLEIC ACIDS RESEARCH, Bd. 22, Nr. 15, 1994, Seiten 2990-2997, XP002210107 ISSN: 0305-1048
- GRUNAU ET AL: "Bisulfite genomic sequencing: systematic investigation of critical experimental parameters" NUCLEIC ACIDS REASEARCH, Bd. 29, Nr. 13, 2001, Seite E65,

### SEQUENZPROTOKOLL

<110> Epigenomics AG
<120> Verfahren zum Nachweis von Cytosin-Methylierungen
<130> E01/1204/WO
<140>
   <141>
<160> 2
<170> PatentIn Ver. 2.1
<210> 1
   <211> 28
   <212> DNA
   <213> Künstliche Sequenz
<220>
   <223> Beschreibung der künstlichen Sequenz: Primer
<400> 1
   agggagtttt ttttagggaa tagaggga 28
<210> 2
   <211> 28
   <212> DNA
   <213> Künstliche Sequenz
<220>
   <223> Beschreibung der künstlichen Sequenz:Primer
<400> 2
   taatcccaaa acctctccac tacaacaa 28

## Beschreibung

Die vorliegende Erfindung betrifft ein Verfahren zum Nachweis von Cytosin-Methylierungen in DNA.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die inzwischen am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z. B. Zechnigk, M. et al., Eur. J. Hum. Gen. 1997, 5, 94-98) nur in der Forschung angewendet. Immer aber werden kurze, spezifische Stücke eines bekannten Gens nach einer Bisulfit-Behandlung amplifziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO-Patent 9500669) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO 99 28498).

Weitere Publikationen, die sich mit der Anwendung der Bisulfit-Technik zum Methylierungsnachweis bei einzelnen Genen befassen, sind: Xiong, Z. und Laird, P. W. (1997), Nucl. Acids Res. 25, 2532; Gonzalgo, M. L. und Jones, P. A. (1997), Nucl. Acids Res. 25, 2529; Grigg, S. und Clark, S. (1994), Bioassays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO 97 46705, WO 95 15373 und WO 45560.

Eine Übersicht über den Stand der Technik in der Oligomer Array Herstellung läßt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonukleotide bei vermindertem nichtspezifischem Hintergrundsignal entnehmen.

Für die Abtastung eines immobilisierten DNA-Arrays sind vielfach fluoreszent markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas, M. und Hillenkamp, F. (1988), Laser desorption ionization of proteins with molecular masses exeeding 10000 daltons. Anal. Chem. 60: 2299-2301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995)), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlererweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

Harnstoff verbessert die Effizienz der Bisulfit-Behandlung vor der Sequenzierung von 5-Methylcytosin in genomischer DNA (Paulin R, Grigg GW, Davey MW, Piper AA.(1998), Nucleic Acids Res. 26: 5009-5010).

Clark et al. ("High sensitivity mapping of methylated cytosines", Nucleic Acids Research, 1994, Bd. 22, Nr. 15, Seiten 2990 - 2997) beschreiben ein Verfahren zur Analyse von methylierten Cytosinen. Sie beschreiben dabei einen Desulphonierungsschritt und eine PCR-Amplifizierung.

Eine Methylierungs-Analyse unter Verwendung einer Bisulphit-Behandlung und einer PCR-Amplifizierung mit einer Mischung von degenerierten Primern wird von Wong et al. ("p16-INK4a promoter is hypermethylated at a high frequency in esophageal adenocarcinomas", Cancer Research, 1997, Bd. 57, Nr. 13, Seiten 2619 - 2622) beschreiben.

Ferner wird in der US 4,851,331 ein Verfahren zur Bestimmung einer Nukleotidsequenz in einer Nukleinsäure einer biologischen Probe und ein dazugehöriges Kit beschrieben.

Des Weiteren beschreiben Landegren et al. ("A ligasemediated gene detection technique", Science, 1988, Bd. 241, Nr. 4869, Seiten 1077 - 1080) ein Verfahren zur Gen-Detektion.

Die Möglichkeiten und Grenzen von modifizierten Ligase-Kettenreakationen bei der Detektion und Analyse von DNA, die sich in einer Base unterscheiden, wurden von Abravaya et al. ("Detection of point mutation with a modified ligase chain reaction (Gap-LCR)", Nucleic Acids Research, 1995, Bd. 23, Nr. 4, Seiten 675 - 682) untersucht.

Aufgabe der vorliegende Erfindung ist es daher eine Verfahren zum Nachweis von Cytosin-Methylierungen in DNA zur Verfügung zu stellen, welches die Nachteile des Standes der Technik überwindet.

Die Aufgabe wird dadurch gelöst, dass ein Verfahren zum Nachweis von Cytosin-Methylierungen in.DNA zur Verfügung gestellt wird, wobei man folgende Arbeitsschritte aus-führt:
a) eine genomische DNA-Probe wird mit einer Lösung eines Bisulfits (= Hydrogensulfit, Disulfit) im Konzentrationsbereich zwischen 0,1 und 6 mol/l inkubiert, wobei ein denaturierendes Lösemittel sowie mindestens ein Radikalfänger zugegen ist;
b) die behandelte DNA-Probe wird mit Wasser oder einer wässrigen Lösung verdünnt;
c) die DNA-Probe wird in einer Polymerasereaktion amplifiziert;
d) wobei man vor Schritt c) eine Desulfonierung der DNA durchführt;
e) man detektiert, inwieweit sich die Sequenz durch die Behandlung nach Schritt a) gegenüber der genomischen DNA-Probe verändert hat und schließt auf den Methylierungsstatus zumindest eines Locus in der genomischen DNA-Probe.

Erfindungsgemäß bevorzugt ist es dabei, dass das denaturierende Reagenz und/oder Lösungsmittel aus der folgenden Liste von Verbindungen oder Verbindungsklassen ausgewählt ist:
Polyethylenglykoldialkylether, Dioxan und substituierte Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO, THF.

Bevorzugt ist dabei ferner, dass der Radikalfänger aus der folgenden Gruppe von Verbindungen ausgewählt ist:
Di-, Trihydroxybenzole, Grüntee Extrakt (green tea extract), Pycnogenol (pine bark extract), Ginkgo Biloba Extrakt (EGb 761), Flavonoid-Mischung verschiedener Frucht- und Gemüseextrakte (GNLD), Bio-Normalizer (Sun-O Corp), DPPH (1,1-Diphenyl-2-picrylhydrazyl), NDGA (Nordihydroguajaret-säure), Trolox (6-Hydroxy-2,5,7,8-tetramethylchroman-2-karbonsäure), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl-germanium-sesquioxid, Superoxid-Dismutase (SOD), Superoxid-Katalase, Alpha-Naphthoflavon, Di(2-methyl-5-chlorophenyl)dithionat und Cu(II)-Derivate, Mebendazol, CS (Chloroformlöslicher) Alkaloid-Extrakt,
4-(3,5-Di-tert-butyl-4-hydroxyphenyl)-3-hydroxy-1,2-naphthochinon,
4-(3,5-Di-tert-butyl-4-hydroxyphenyl)-3-methoxy-1,2-naphthochinon,
4-(3,5-Di-tert-butyl-4-hydroxyphenyl)-1,2-naphthochinon,
2-(3,5-Di-tert-butyl-4-hydroxyphenyl)-3-brom-1,4-naphthochinon,
2-(3,5-Di-tert-butyl-4-hydroxyphenyl)-3-chlor-1,4-naphthochinon,
2-(3,5-Di-tert-butyl-4-hydroxyphenyl)-3-methoxy-1,4-naphthochinon,
2-(3,5-Di-tert-butyl-4-hydroxyphenyl)-3-hydroxy-1,4-naphthochinon,
2-(3,5-Di-tert-butyl-4-hydroxyphenyl)-1,4-naphthochinon,
4-(3,5-Di-tert-butyl-4-hydroxyphenyl)-3-hydroxy-5,5,8,8-tetramethyl-5,6,7,8-tetrahydro-1,2-anthrachinon,
4-(3,5-Di-tert-butyl-4-hydroxyphenyl)-3-methoxy-5,5,8,8-tetramethyl-5,6,7,8-tetrahydro-1,2-anthrachinon,
4-(3,5-Di-tert-butyl-4-hydroxyphenyl)-5,5,8,8-tetramethyl-5,6,7,8-tetrahydro-1,2-anthrachinon,
3-Brom-4-(3,5-di-tert-butyl-4-hydroxyphenyl)-5,5,8,8-tetramethyl-5,6,7,8-tetrahydro-1,2-anthrachinon,
2-(3,5-Di-tert-butyl-4-oxocyclohexa-2,5-dienyliden)-indan-1,3-dion,
2-(3,5-Di-tert-butyl-4-oxocyclohexa-2,5-dienyliden)-3,4-epoxy-3-hydroxy-4-methoxy-3,4-dihydro-2H-naphthalin-1-on,
2-(3,5-Di-tert-butyl-4-oxocyclohexa-2,5-dienyliden)-3,4-epoxy-3,4-dimethoxy-3,4-dihydro-2H-naphthalin-1-on,
2-(3,5-Di-tert-butyl-4-hydroxyphenyl)-indan-1-on, 3,3-Bi-[2-(3,5-di-tert-butyl-4-hydroxyphenyl)-inden-1-on]-3-yl,
2-(3,5-Di-tert-butyl-4-hydroxyphenyl)-3-brom-5,5,8,8-tetramethyl-5,6,7,8-tetrahydro-1,4-anthrachinon,
2-(3,5-Di-tert-butyl-4-hydroxyphenyl)-3-chlor-5,5,8,8-tetramethyl-5,6,7,8-tetrahydro-1,4-anthrachinon,
2-(3,5-Di-tert-butyl-4-hydroxyphenyl)-3-methoxy-5,5,8,8-tetramethyl-5,6,7,8-tetrahydro-1,4-anthrachinon,
2-(3,5-Di-tert-butyl-4-hydroxyphenyl)-3-hydroxy-5,5,8,8-tetramethyl-5,6,7,8-tetrahydro-1,4-anthrachinon,
2-(3,5-Di-tert-butyl-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-5,5,8,8-tetramethyl-5,6,7,8-tetrahydro-1,4-anthrachinon,
2-Brom-3-(3,5-dibrom-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-3-hydroxy-5,5,8,8-tetramethyl-5,6,7,8-tetrahydro-1,4-anthrachinon,
3-Brom-2-(3,5-di-tert-butyl-4-hydroxyphenyl)-1,4-anthrachinon,
2-(3,5-Di-tert-butyl-4-hydroxyphenyl)-3-methoxy-1,4-anthrachinon,
2-(3,5-Di-tert-butyl-4-hydroxyphenyl)-3-hydroxy-1,4-anthrachinon,
5,5,8,8-Tetramethyl-5,6,7,8-tetrahydronaphthalin-1,3-diol,
3-Methoxy-5,5,8,8-tetramethyl-5,6,7,8-tetrahydronaphthalin-1-ol,
4-(3-Chlor-5,5,8,8-tetramethyl-1,4-dioxo-1,4,5,6,7,8-hexahydroanthracen-2-yl)-benzoesäure,
Methyl-4-(3-chlor-5,5,8,8-tetramethyl-1,4-dioxo-1,4,5,6,7,8-hexahydroanthracen-2-yl)-benzoat,
4-(3-Hydroxy-1,4-dioxo-1,4-dihydronaphthalin-2-yl)-benzoesäure,
Methyl-(3-methoxy-1,4-dioxo-1,4-dihydronaphthalin-2-yl)-benzoesäure,
4-(3-Hydroxy-5,5,8,8-tetramethyl-1,4-dioxo-1,4,5,6,7,8-hexahydroanthracen-2-yl)-benzoesäure,
Methyl-4-(3-hydroxy-1,4-dioxo-1,4-dihydronaphthalin-2-yl-azo)-benzoat,
4-(3-Hydroxy-5,5,8,8-tetramethyl-1,4-dioxo-1,4,5,6,7,8-hexahydroanthracen-2-yl-azo)-benzoesäure,
3-(3,5-Di-tert-butyl-4-oxocyclohexa-2,5-dienyliden)-5, 5, 8, 8-tetramethyl-5, 6, 7, 8-tetrahydrocyclopenta[b]naphthalin-1,2-dion,
3-(3,5-Di-tert-butyl-4-oxocyclohexa-2,5-dienyliden)-5,5,8,8-tetramethyl-5,6,7,8-tetrahydroanthracen-3H-1,2,4-trion,
2-(3,5-Di-tert-butyl-4-hydroxyphenyl)-3-methoxy-5,8-dimethyl-1,4-naphthochinon,
2-(3,5-Di-tert-butyl-4-hydroxyphenyl)-3-methoxy-6,7-dimethyl-1,4-naphthochinon,
2-(3,5-Di-tert-butyl-4-hydroxyphenyl)-3-methoxy-5-methyl-1,4-naphthochinon,
2-(3,5-Di-tert-butyl-4-hydroxyphenyl)-2-methoxy-5-methyl-1,4-naphthochinon,
2-(3,5-Di-tert-butyl-4-hydroxyphenyl)-3-methoxy-6-methyl-1,4-naphthochinon,
3-(3,5-Di-tert-butyl-4-hydroxyphenyl)-2-methoxy-6-methyl-1,4-naphthochinon,
2-(3,5-Di-tert-butyl-4-hydroxyphenyl)-3-methoxy-5,6-dimethyl-1,4-naphthochinon,
3-(3,5-Di-tert-butyl-4-hydroxyphenyl)-2-methoxy-5,6-dimethyl-1,4-naphthochinon,
2-(3,5-Di-tert-butyl-4-hydroxyphenyl)-3-methoxy-5,7-dimethyl-1,4-naphthochinon,
3-(3,5-Di-tert-butyl-4-hydroxyphenyl)-2-methoxy-5,7-dimethyl-1,4-naphthochinon,
2-(3,5-Di-tert-butyl-4-hydroxyphenyl)-3-ethylthio-5-methyl-1,4-naphthochinon,
2-(3,5-Di-tert-butyl-4-hydroxyphenyl)-3-ethylthio-6-methyl-1,4-naphthochinon,
2-(3,5-Di-tert-butyl-4-hydroxyphenyl)-3-hydroxy-5,8-dimethyl-1,4-naphthochinon,
2-(3,5-Di-tert-butyl-4-hydroxyphenyl)-3-hydroxy-6,7-dimethyl-1,4-naphthochinon,
2-(3,5-Di-tert-butyl-4-hydroxyphenyl)-3-hydroxy-5-methyl-1,4-naphthochinon,
3-(3,5-Di-tert-butyl-4-hydroxyphenyl)-2-hydroxy-5-methyl-1,4-naphthochinon,
2-(3,5-Di-tert-butyl-4-hydroxyphenyl)-3-hydroxy-6-methyl-1,4-naphthochinon,
3-(3,5-Di-tert-butyl-4-hydroxyphenyl)-2-hydroxy-6-methyl-1,4-naphthochinon,
2-(3,5-Di-tert-butyl-4-hydroxyphenyl)-3-hydroxy-5,6-dimethyl-1,4-naphthochinon,
2-(3-Brom-5-tert-butyl-4-hydroxyphenyl)-3-hydroxy-5,6-dimethyl-1,4-naphthochinon,
3-(3,5-Di-tert-butyl-4-hydroxyphenyl)-2-hydroxy-5,6-dimethyl-1,4-naphthochinon,
2-(3,5-Di-tert-butyl-4-hydroxyphenyl)-3-hydroxy-5,7-dimethyl-1,4-naphthochinon,
3-(3,5-Di-tert-butyl-4-hydroxyphenyl)-2-hydroxy-5,7-dimethyl-1,4-naphthochinon.

Erfindungsgemäß bevorzugt ist dabei, dass man die genomische DNA-Probe vor der Behandlung thermisch denaturiert.

Besonders erfindungsgemäß bevorzugt ist es, dass man den Schritt c) in zwei Teilschritten wie folgt durchführt: a) eine PCR Präamplifikation mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben; b) eine PCR Amplifikation des in der Präamplifikation gebildeten Produkts mit Primern unterschiedlicher Sequenz, die jeweils zu einem Abschnitt der nach Anspruch 1 vorbehandelten DNA-Probe [(+)-Strang oder (-)-Strang] identisch oder revers komplementär sind und die zu amplifizierende DNA spezifisch hybridisieren.

Es ist erfindungsgemäß weiterhin bevorzugt, dass man die Amplifikation von mehreren DNA-Abschnitten in einem Reaktionsgefäß durchführt.

Bevorzugt ist es erfindungsgemäß außerdem, dass man für die Polymerasereaktion eine hitzebeständige DNA-Polymerase verwendet.

Es ist auch bevorzugt, dass man für die Detektion der vorbehandelten DNA die PCR-Produkte auf einen Oligonukleotid Array hybridisiert und man anschließend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) man das Oligonukleotid mit bekannter Sequenz von n Nukleotiden mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

Es ist erfindungsgemäß bevorzugt, dass man für die Detektion der vorbehandelten DNA die PCR-Produkte auf einen Oligonukleotid Array hybridisiert und man anschließend die folgenden Teilschritte ausgeführt:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) man das Oligonukleotid der ersten Spezies mit bekannter Sequenz von n Nukleotiden mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

Dabei ist es erfindungsgemäß besonders bevorzugt, dass die verwendeten Oligonukleotide der ersten Spezies und/oder die verwendeten Oligonukleotide der zweiten Spezies entweder nur die Basen T, A und C oder aber die Basen T, A und G enthalten.

Es ist außerdem erfindungsgemäß bevorzugt, dass man für die Detektion der vorbehandelten DNA die PCR-Produkte auf einen Oligonukleotid Array hybridisiert und man anschließend die folgenden Teilschritte ausführt:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) man das Oligonukleotid, sofern es mit seinem 3'-Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

Es ist auch erfindungsgemäß bevorzugt, dass man die PCR-Produkte und/oder Verlängerungsprodukte und/oder Ligationsprodukte für die Detektion mit einer nachweisbaren Markierung versieht. Dabei ist es besonders bevorzugt, dass die Markierungen Fluoreszenzmarkierungen und/oder dass die Markierungen Radionuklide sind. Besonders bevorzugt ist es dabei, dass die Markierungen der Nukleotide ablösbare Massenmarkierungen sind, die in einem Massenspektrometer nachweisbar sind.

Insbesondere ist es auch bevorzugt, dass man die PCR-Produkte und/oder Verlängerungsprodukte und/oder Ligationsprodukte insgesamt im Massenspektrometer nachweist und somit durch ihre Masse eindeutig charakterisiert sind. Bevorzugt ist es erfindungsgemäß auch, dass man jeweils ein Fragment der PCR-Produkte und/oder Verlängerungsprodukte und/oder Ligationsprodukte im Massenspektrometer nachweist.

Das erfindungsgemäße Verfahren ist bevorzugt auch dadurch gekennzeichnet, dass man das Fragment des PCR-Produkts und/oder Verlängerungsprodukts und/oder Ligationsprodukts durch Verdau mit einer oder mehrerer Exo- oder Endonukleasen erzeugt.

Es ist weiterhin bevorzugt, dass man zur besseren Detektierbarkeit im Massenspektrometer die erzeugten Fragmente mit einer einzelnen positiven oder negativen Nettoladung versieht.

Ganz besonders bevorzugt ist es, dass man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

Das erfindungsgemäße Verfahren ist auch derart bevorzugt, in dem man die genomische DNA aus einer DNA-Probe erhält, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Ein weiterer Gegenstand der vorliegenden Erfindung ist die Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen: Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Gegenstand der Erfindung ist auch die Verwendung eines erfindungsgemäßen Verfahrens zur Unterscheidung von Zelltypen oder Geweben oder zur Untersuchung der Zelldifferenzierung.

Ein weiterer Gegenstand der vorliegenden Erfindung ist schließlich ein Kit, bestehend aus einem Bisulfit enthaltenen Reagenz, denaturierenden Lösungsmitteln, sowie Radikalfängern und Primern zur Herstellung der Amplifikate, sowie eine Anleitung zur Durchführung eines Assays nach einem erfindungsgemäßen Verfahren.

Die vorliegende Erfindung stellt ein automatisierbares Verfahren zum Nachweis von Methylcytosin bereit, welches nur Pipettierschritte enthält. Dadurch wird die Effizienz bestehender Verfahren in Bezug auf die Einfachheit der Handhabung, die Qualität, die Kosten und vor allem den Durchsatz verbessert.

Beschrieben wird ein automatisierbares Verfahren zum Nachweis von Methylcytosin in genomischen DNA-Proben:

Die zu analysierende genomische DNA wird bevorzugt aus den üblichen Quellen für DNA erhalten, wie z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon.

Im ersten Schritt des Verfahrens behandelt man die eingesetzte DNA bevorzugt mit Bisulfit, (= Disulfit, Hydrogensulfit) derart, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben.

Die genomische DNA-Probe denaturiert man besonders bevorzugt vor der Behandlung thermisch.

Wird für die Reaktion Bisulfit im Konzentrationsbereich zwischen 0.1 und 6 mol/l verwendet, so findet an den nicht methylierten Cytosinbasen eine Addition statt. Für das erfindungsgemäße Verfahren müssen zudem ein denaturierendes Reagenz oder Lösungsmittel sowie ein Radikalfänger zugegen sein.

Dabei kommen als denaturierende Reagenzien oder Lösungsmittel bevorzugt die folgenden Verbindungen oder Verbindungsklassen in Frage:

Polyethylenglykoldialkylether, Dioxan und substituierte Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO oder THF.

Als Radikalfänger ist die Gruppe der in Liste 1 aufgelisteten Verbindungen oder deren Derivate vorzugsweise geeignet. Die anschließende alkalische Hydrolyse führt dann zur Umwandlung von nicht methylierten Cytosin-Nukleobasen in Uracil.

Im zweiten Verfahrensschritt verdünnt man die behandelte DNA-Probe mit Wasser oder einer wässrigen Lösung. Bevorzugt wird anschließend eine Desulfonierung der DNA (10-30 min, 90-100 °C) bei alkalischem pH-Wert durchgeführt.

Im dritten Schritt des Verfahrens amplifiziert man die DNA-Probe in einer Polymerasekettenreaktion, bevorzugt mit einer hitzebeständigen DNA-Polymerase. Die Amplifikation von mehreren DNA-Abschnitten wird vorzugsweise in einem Reaktionsgefäß gemacht.

Den Verfahrensschritt führt man vorzugsweise in zwei Teilschritten durch. Man beginnt mit einer PCR Präamplifikation mit mindestens einem Primerpaar unterschiedlicher Sequenz, welche die vorbehandelte DNA Probe unspezifisch hybridisieren und daher im PCR Schritt mehr als ein Amplifkat ergeben. Danach führt man eine PCR Amplifikation des in der Präamplifikation gebildeten Produkts mit Primern unterschiedlicher Sequenz durch, die jeweils zu einem Abschnitt der vorbehandelten DNA-Probe [(+)-Strang oder (-)-Strang] identisch oder revers komplementär sind und die zu amplifizierende DNA spezifisch hybridisieren.

Dabei ist es klar, dass derartige Präamplifikationen häufig nicht als PCR Reaktionen, sondern als Primerextensionsreaktionen ausgeführt werden, die keine hitzebeständige Polymerase erfordern.

Im letzten Verfahrensschritt detektiert man, inwieweit sich die Sequenz durch die Behandlung mit einem Bisulfit enthaltenen Reagenz gegenüber der genomischen DNA-Probe verändert hat und schließt auf den Methylierungsstatus zumindest eines Locus in der genomischen DNA-Probe.

Für die Detektion werden die PCR-Produkte besonders bevorzugt auf einen Oligonukleotid Array hybridisiert.

In einer bevorzugten Variante des Verfahrens führt man nach der Hybridisierung auf einen Oligonukleotid Array die folgenden Teilschritte durch:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) das Oligonukleotid mit bekannter Sequenz von n Nukleotiden wird mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

In einer weiteren bevorzugten Variante des Verfahrens führt man nach der Hybridisierung auf einen Oligonukleotid Array die folgenden Teilschritte durch:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) das Oligonukleotid der ersten Spezies mit bekannter Sequenz von n Nukleotiden wird mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt, welches bevorzugt phosphoryliert vorliegt.

Die verwendeten Oligonukleotide der ersten Spezies und/oder die verwendeten Oligonukleotide der zweiten Spezies enthalten besonders bevorzugt entweder nur die Basen T, A und C oder aber die Basen T, A und G.

In einer wiederum bevorzugten Variante des Verfahrens führt man nach der Hybridisierung auf einen Oligonukleotid Array die folgenden Teilschritte durch:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) das Oligonukleotid wird, sofern es mit seinem 3'Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

Die PCR-Produkte und/oder Verlängerungsprodukte und/oder Ligationsprodukte sind für die Detektion besonders bevorzugt mit einer nachweisbaren Markierung versehen.

Vorzugsweise sind die Markierungen der PCR-Produkte und/oder Verlängerungsprodukte und/oder Ligationsprodukte Fluoreszenzmarkierungen, Radionuklide oder ablösbare Massenmarkierungen, die in einem Massenspektrometer nachgewiesen werden.

Die PCR-Produkte und/oder Verlängerungsprodukte und/oder Ligationsprodukte können vorzugsweise insgesamt im Massenspektrometer nachgewiesen werden und sind somit durch ihre Masse eindeutig charakterisiert.

Besonders bevorzugt weist man jeweils ein Fragment der PCR-Produkte und/oder Verlängerungsprodukte und/oder Ligationsprodukte im Massenspektrometer nach.

Das Fragment des PCR-Produkts und/oder Verlängerungsprodukts und/oder Ligationsprodukts erzeugt man bevorzugt durch Verdau mit einer oder mehrerer Exo- oder Endonukleasen.

Zur besseren Detektierbarkeit im Massenspektrometer weisen die erzeugten Fragmente besonders bevorzugt eine einzelne positive oder negative Nettoladung auf.

Die PCR-Produkte und/oder Verlängerungsprodukte und/oder Ligationsprodukte detektiert und visualisiert man vorzugsweise mittels Matrix assistierter Laser Desorptions/Ionisations Massenspektrometrie (MALDI-TOF) oder mittels Elektrospray Massenspektrometrie (ESI).

Das vorliegende Verfahren wird bevorzugt verwendet zur Diagnose und/oder Prognose von nachteiligen Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Das neue Verfahren dient ferner besonders bevorzugt zur Unterscheidung von Zelltypen, Geweben oder zur Untersuchung der Zelldifferenzierung.

Gegenstand der vorliegenden Erfindung ist ferner ein Kit, das ein Bisulfit enthaltenes Reagenz, denaturierende Lösungsmittel, sowie Radikalfänger gemäss Liste 1, Primer zur Herstellung der Amplifikate und eine Anleitung zur Durchführung eines Assays enthält.

### Liste 1:

Di-, Trihydroxybenzole, Grüntee Extrakt (green tea extract), Pycnogenol (pine bark extract), Ginkgo Biloba Extrakt (EGb 761), Flavonoid-Mischung verschiedener Frucht- und Gemüseextrakte (GNLD), Bio-Normalizer (Sun-O Corp),
DPPH (1,1-Diphenyl-2-picrylhydrazyl), NDGA (Nordihydroguajaret-säure),
Trolox (6-Hydroxy-2,5,7,8-tetramethylchroman-2-karbonsäure),
2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl-germanium-sesquioxid, Superoxid-Dismutase (SOD), Superoxid-Katalase, Alpha-Naphthoflavon, , Di(2-methyl-5-chlorophenyl)dithionat und Cu(II)-Derivate, Mebendazol, CS (Chloroformlöslicher) Alkaloid-Extrakt,
4-(3,5-Di-tert-butyl-4-hydroxyphenyl)-3-hydroxy-1,2-naphthochinon,
4-(3,5-Di-tert-butyl-4-hydroxyphenyl)-3-methoxy-1,2-naphthochinon,
4-(3,5-Di-tert-butyl-4-hydroxyphenyl)-1,2-naphthochinon,
2-(3,5-Di-tert-butyl-4-hydroxyphenyl)-3-brom-1,4-naphthochinon,
2-(3,5-Di-tert-butyl-4-hydroxyphenyl)-3-chlor-1,4-naphthochinon,
2-(3,5-Di-tert-butyl-4-hydroxyphenyl)-3-methoxy-1,4-naphthochinon,
2-(3,5-Di-tert-butyl-4-hydroxyphenyl)-3-hydroxy-1,4-naphthochinon,
2-(3,5-Di-tert-butyl-4-hydroxyphenyl)-1,4-naphthochinon,
4-(3,5-Di-tert-butyl-4-hydroxyphenyl)-3-hydroxy-5,5,8,8-tetramethyl-5,6,7,8-tetrahydro-1,2-anthrachinon,
4-(3,5-Di-tert-butyl-4-hydroxyphenyl)-3-methoxy-5,5,8,8-tetramethyl-5,6,7,8-tetrahydro-1,2-anthrachinon,
4-(3,5-Di-tert-butyl-4-hydroxyphenyl)-5,5,8,8-tetramethyl-5,6,7,8-tetrahydro-1,2-anthrachinon,
3-Brom-4-(3,5-di-tert-butyl-4-hydroxyphenyl)-5,5,8,8-tetramethyl-5,6,7,8-tetrahydro-1,2-anthrachinon,
2-(3,5-Di-tert-butyl-4-oxocyclohexa-2,5-dienyliden)-indan-1,3-dion,
2-(3,5-Di-tert-butyl-4-oxocyclohexa-2,5-dienyliden)-3,4-epoxy-3-hydroxy-4-methoxy-3,4-dihydro-2H-naphthalin-1-on,
2-(3,5-Di-tert-butyl-4-oxocyclohexa-2,5-dienyliden)-3,4-epoxy-3,4-dimethoxy-3,4-dihydro-2H-naphthalin-1-on,
2-(3,5-Di-tert-butyl-4-hydroxyphenyl)-indan-1-on, 3,3-Bi-[2-(3,5-di-tert-butyl-4-hydroxyphenyl)-inden-1-on]-3-yl,
2-(3,5-Di-tert-butyl-4-hydroxyphenyl)-3-brom-5,5,8,8-tetramethyl-5,6,7,8-tetrahydro-1,4-anthrachinon,
2-(3,5-Di-tert-butyl-4-hydroxyphenyl)-3-chlor-5,5,8,8-tetramethyl-5,6,7,8-tetrahydro-1,4-anthrachinon,
2-(3,5-Di-tert-butyl-4-hydroxyphenyl)-3-methoxy-5,5,8,8-tetramethyl-5,6,7,8-tetrahydro-1,4-anthrachinon,
2-(3,5-Di-tert-butyl-4-hydroxyphenyl)-3-hydroxy-5,5,8,8-tetramethyl-5,6,7,8-tetrahydro-1,4-anthrachinon,
2-(3,5-Di-tert-butyl-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-5,5,8,8-tetramethyl-5,6,7,8-tetrahydro-1,4-anthrachinon,
2-Brom-3-(3,5-dibrom-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-3-hydroxy-5,5,8,8-tetramethyl-5,6,7,8-tetrahydro-1,4-anthrachinon,
3-Brom-2-(3,5-di-tert-butyl-4-hydroxyphenyl)-1,4-anthrachinon,
2-(3,5-Di-tert-butyl-4-hydroxyphenyl)-3-methoxy-1,4-anthrachinon,
2-(3,5-Di-tert-butyl-4-hydroxyphenyl)-3-hydroxy-1,4-anthrachinon,
5,5,8,8-Tetramethyl-5,6,7,8-tetrahydronaphthalin-1,3-diol,
3-Methoxy-5,5,8,8-tetramethyl-5,6,7,8-tetrahydronaphthalin-1-ol,
4-(3-Chlor-5,5,8,8-tetramethyl-1,4-dioxo-1,4,5,6,7,8-hexahydroanthracen-2-yl)-benzoesäure,
Methyl-4-(3-chlor-5,5,8,8-tetramethyl-1,4-dioxo-1,4,5,6,7,8-hexahydroanthracen-2-yl)-benzoat,
4-(3-Hydroxy-1,4-dioxo-1,4-dihydronaphthalin-2-yl)-benzoesäure,
Methyl-(3-methoxy-1,4-dioxo-1,4-dihydronaphthalin-2-yl)-benzoesäure,
4-(3-Hydroxy-5,5,8,8-tetramethyl-1,4-dioxo-1,4,5,6,7,8-hexahydroanthracen-2-yl)-benzoesäure,
Methyl-4-(3-hydroxy-1,4-dioxo-1,4-dihydronaphthalin-2-yl-azo)-benzoat,
4-(3-Hydroxy-5,5,8,8-tetramethyl-1,4-dioxo-1,4,5,6,7,8-hexahydroanthracen-2-yl-azo)-benzoesäure,
3-(3,5-Di-tert-butyl-4-oxocyclohexa-2,5-dienyliden)-5,5,8,8-tetramethyl-5,6,7,8-tetrahydrocyclopenta[b]naphthalin-1,2-dion,
3-(3,5-Di-tert-butyl-4-oxocyclohexa-2,5-dienyliden)-5,5,8,8-tetramethyl-5,6,7,8-tetrahydroanthracen-3H-1,2,4-trion,
2-(3,5-Di-tert-butyl-4-hydroxyphenyl)-3-methoxy-5,8-dimethyl-1,4-naphthochinon,
2-(3,5-Di-tert-butyl-4-hydroxyphenyl)-3-methoxy-6,7-dimethyl-1,4-naphthochinon,
2-(3,5-Di-tert-butyl-4-hydroxyphenyl)-3-methoxy-5-methyl-1,4-naphthochinon,
2-(3,5-Di-tert-butyl-4-hydroxyphenyl)-2-methoxy-5-methyl-1,4-naphthochinon,
2-(3,5-Di-tert-butyl-4-hydroxyphenyl)-3-methoxy-6-methyl-1,4-naphthochinon,
3-(3,5-Di-tert-butyl-4-hydroxyphenyl)-2-methoxy-6-methyl-1,4-naphthochinon,
2-(3,5-Di-tert-butyl-4-hydroxyphenyl)-3-methoxy-5,6-dimethyl-1,4-naphthochinon,
3-(3,5-Di-tert-butyl-4-hydroxyphenyl)-2-methoxy-5,6-dimethyl-1,4-naphthochinon,
2-(3,5-Di-tert-butyl-4-hydroxyphenyl)-3-methoxy-5,7-dimethyl-1,4-naphthochinon,
3-(3,5-Di-tert-butyl-4-hydroxyphenyl)-2-methoxy-5,7-dimethyl-1,4-naphthochinon,
2-(3,5-Di-tert-butyl-4-hydroxyphenyl)-3-ethylthio-5-methyl-1,4-naphthochinon,
2-(3,5-Di-tert-butyl-4-hydroxyphenyl)-3-ethylthio-6-methyl-1,4-naphthochinon,
2-(3,5-Di-tert-butyl-4-hydroxyphenyl)-3-hydroxy-5,8-dimethyl-1,4-naphthochinon,
2-(3,5-Di-tert-butyl-4-hydroxyphenyl)-3-hydroxy-6,7-dimethyl-1,4-naphthochinon,
2-(3,5-Di-tert-butyl-4-hydroxyphenyl)-3-hydroxy-5-methyl-1,4-naphthochinon,
3-(3,5-Di-tert-butyl-4-hydroxyphenyl)-2-hydroxy-5-methyl-1,4-naphthochinon,
2-(3,5-Di-tert-butyl-4-hydroxyphenyl)-3-hydroxy-6-methyl-1,4-naphthochinon,
3-(3,5-Di-tert-butyl-4-hydroxyphenyl)-2-hydroxy-6-methyl-1,4-naphthochinon,
2-(3,5-Di-tert-butyl-4-hydroxyphenyl)-3-hydroxy-5,6-dimethyl-1,4-naphthochinon,
2-(3-Brom-5-tert-butyl-4-hydroxyphenyl)-3-hydroxy-5,6-dimethyl-1,4-naphthochinon,
3-(3,5-Di-tert-butyl-4-hydroxyphenyl)-2-hydroxy-5,6-dimethyl-1,4-naphthochinon,
2-(3,5-Di-tert-butyl-4-hydroxyphenyl)-3-hydroxy-5,7-dimethyl-1,4-naphthochinon,
3-(3,5-Di-tert-butyl-4-hydroxyphenyl)-2-hydroxy-5,7-dimethyl-1,4-naphthochinon.

Das folgende Beispiel erläutert die Erfindung.

### Beispiel:

### Automatisierte Durchführung der Bisulfitreaktion.

Im vorliegenden Beispiel wird die Anwendung des Verfahrens zum Nachweis des Methylierungsstatus von Cytosinen im Faktor VIII-Gen einer genomischen DNA-Probe, die mit einer Restriktionsendonuclease nach Angabe des Herstellers behandelt wurde, beschrieben. Das Verfahren beruht auf dem Einsatz eines automatischen Pipettiersystems (MWG RoboSeq 4204) mit vier separat vertikal beweglichen Adaptern für austauschbare Pipettierspitzen, die Kreuzkontaminationen ausschließen. Das Pipettiersystem ermöglicht das Pipettieren von 100µl mit einem Fehler von weniger als ±2µl. Die Arbeitsplatte des automatischen Pipettiersystems ist mit sechs Gestellen für Pipettierspitzen und acht Pipettierpositionen, von denen zwei gekühlt werden können, einem kühlbaren Reagenziengestell, einem Stapelsystem für 10 Mikrotiterplatten, einer Pipettierspitzenwaschstation und einer Vorrichtung zur Trennung der Pipettierspitzen vom Adaptor ausgerüstet. Das automatische Pipettiersystem ist über eine serielle Schnittstelle mit einem Computer verbunden und wird über ein Softwareprogramm, das die freie Programmierung aller für zur Anwendung des Verfahrens notwendigen Pipettierschritte erlaubt, gesteuert.

Im ersten Verfahrensschritt wird von Hand ein Aliquot der DNA-Probe in eine von 96 frei wählbaren Positionen einer Mikrotiterplatte pipettiert. Die Mikrotiterplatte wird anschließend unter Verwendung eines Eppendorf Mastercyclers zur Denaturierung der vorbehandelten DNA-Probe auf 96°C erwärmt. Die Mikrotiterplatte wird dann in das automatische Pipettiersystem überführt. In alle Positionen, die DNA enthalten, werden programmgesteuert nacheinander aus dem Reagenziengestell Aliquots eines denaturierenden Agens (Dioxan), einer 3,3 molaren Natriumhydrogensulfitlösung, und einer Lösung eines Radikalfängers in dem verwendeten denaturierenden Agens hinzu pipettiert. Anschließend wird die Mikrotiterplatte im Eppendorf Mastercycler inkubiert, dass in der DNA-Probe unter Einwirkung des Natriumhydrogensulfits alle unmethylierten Cytosinreste in ein Bisulfitaddukt umgewandelt werden.

Nach der Bisulfitbehandlung wird die Mikrotiterplatte aus dem Thermocycler in das automatische Pipetiersystem überführt. Es wird eine zweite Mikrotiterplatte desselben Typs vorgelegt. In alle Kammern, deren äquivalente Position auf der ersten Mikrotiterplatte eine bisulfitbehandelte DNA-Probe enthält, wird zuerst ein basischer Tris-HCl Puffer (pH 9.5) und anschließend wird ein Aliquot der bisulfitbehandelten DNA in die entsprechende Position der zweiten Mikrotiterplatte übertragen. In der basischen Lösung werden die Bisulfitaddukte der nichtmethylierten Cytosinreste zu Uracilresten umgewandelt.

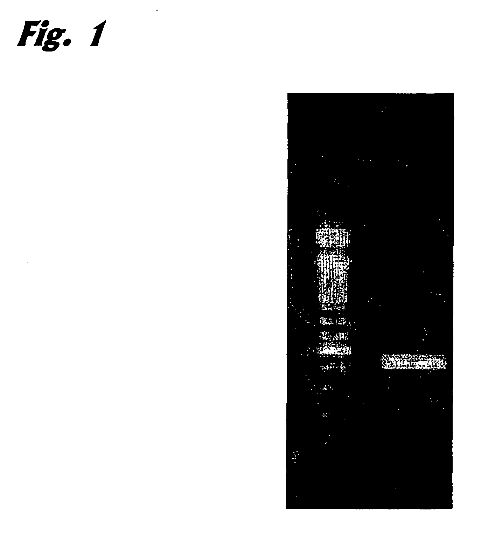

Die gezielte Amplifikation eines Stranges (im vorliegenden Beispiel der sense-Strang) der bisulfitbehandelten DNA erfolgt durch eine Polymerasekettenreaktion (PCR). Es wird ein Primerpaar des Typs 1 (AGG GAG TTT TTT TTA GGG AAT AGA GGG A (SEQ-ID:1) und TAA TCC CAA AAC CTC TCC ACT ACA ACA A (SEQ-ID:2)) verwendet, das die spezifische Amplifikation eines erfolgreich bisulfitbehandelten DNA-Strangs, nicht jedoch eines DNA-Strangs, dessen nichtmetylierte Cytosinreste nicht oder unvollständig in Uracilreste umgewandelt wurden, erlaubt. Für die PCR-Reaktion wird im automatischen Pipettiersystem eine dritte Mikrotiterplatte desselben Typs vorgelegt. In alle Kammern, deren äquivalente Positionen auf der ersten Mikrotiterplatte eine bisulfitbehandelte DNA-Probe enthält, wird zuerst ein Aliquot einer Stammlösung, die einen PCR-Puffer, eine DNA-Polymerase und Primer des Typs 1 enthält, automatisch pipettiert. Danach wird automatisch aus jeder Position der zweiten Mikrotiterplatte ein Aliquot der verdünnten bisulfitbehandelten DNA in die entsprechende Position der dritten Mikrotiterplatte übertragen, bevor diese zur Durchführung der PCR-Reaktion in den Cycler überführt wird. Das PCR-Produkt wird durch Agarosegelelektrophorese und anschließende Anfärbung mit Ethidiumbromid identifiziert (Fig. 1). Figur 1 zeigt das Gelbild eines PCR-amplifizierten bisulfitbehandelten DNA-Stranges (links: Molekulargewichtsmarker, rechts: PCR-Produkt)

## Patentansprüche

1. Verfahren zum Nachweis von Cytosin-Methylierungen in DNA, **dadurch gekennzeichnet, dass** man folgende Arbeitsschritte ausführt:
a) eine genomische DNA-Probe wird mit einer Lösung eines Bisulfits (= Hydrogensulfit, Disulfit) im Konzentrationsbereich zwischen 0,1 und 6 mol/l inkubiert, wobei ein denaturierendes Lösemittel sowie mindestens ein Radikalfänger zugegen ist;
b) die behandelte DNA-Probe wird mit Wasser oder einer wässrigen Lösung verdünnt;
c) die DNA-Probe wird in einer Polymerasereaktion amplifiziert;
d) wobei man vor Schritt c) eine Desulfonierung der DNA durchführt;
e) man detektiert, inwieweit sich die Sequenz durch die Behandlung nach Schritt a) gegenüber der genomischen DNA-Probe verändert hat und schliesst auf den Methylierungsstatus zumindest eines Locus in der genomischen DNA-Probe.

2. Verfahren nach Anspruch 1, **dadurch gekennzeichnet, dass** das denaturierende Reagenz und/oder Lösungsmittel aus der folgenden Liste von Verbindungen oder Verbindungsklassen ausgewählt ist:
Polyethylenglykoldialkylether, Dioxan und substituierte Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO, THF.

3. Verfahren nach Anspruch 1 oder 2, **dadurch gekennzeichnet, dass** der Radikalfänger aus der folgenden Gruppe von Verbindungen ausgewählt ist :
Di-, Trihydroxybenzole, Grüntee Extrakt (green tea extract), Pycnogenol (pine bark extract), Ginkgo Biloba Extrakt (EGb 761), Flavonoid-Mischung verschiedener Frucht- und Gemüseextrakte (GNLD), Bio-Normalizer (Sun-O Corp), DPPH (1,1-Diphenyl-2-picrylhydrazyl), NDGA (Nordihydroguajaret-säure), Trolox (6-Hydroxy-2,5,7,8-tetramethylchroman-2-karbonsäure), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl-germanium-sesquioxid, Superoxid-Dismutase (SOD), Superoxid-Katalase, Alpha-Naphthoflavon, Di(2-methyl-5-chlorophenyl)dithionat und Cu(II)-Derivate, Mebendazole, CS (Chloroformlöslicher) Alkaloid-Extrakt,
4-(3,5-Di-tert-butyl-4-hydroxyphenyl)-3-hydroxy-1,2-naphthochinon,
4-(3,5-Di-tert-butyl-4-hydroxyphenyl)-3-methoxy-1,2-naphthochinon,
4-(3,5-Di-tert-butyl-4-hydroxyphenyl)-1,2-naphthochinon,
2-(3,5-Di-tert-butyl-4-hydroxyphenyl)-3-brom-1,4-naphthochinon,
2-(3,5-Di-tert-butyl-4-hydroxyphenyl)-3-chlor-1,4-naphthochinon,
2-(3,5-Di-text-butyl-4-hydroxyphenyl)-3-methoxy-1,4-naphthochinon,
2-(3,5-Di-tert-butyl-4-hydroxyphenyl)-3-hydroxy-1,4-naphthochinon,
2-(3,5-Di-tert-butyl-4-hydroxyphenyl)-1,4-naphthochinon,
4-(3,5-Di-tert-butyl-4-hydroxyphenyl)-3-hydroxy-5,5,8,8-tetramethyl-5,6,7,8-tetrahydro-1,2-anthrachinon,
4-(3,5-Di-tert-butyl-4-hydroxyphenyl)-3-methoxy-5,5,8,8-tetramethyl-5,6,7,8-tetrahydro-1,2-anthrachinon,
4- (3, 5-Di-tert-butyl-4-hydroxyphenyl)-5,5,8,8-tetramethyl-5,6,7,8-tetrahydro-1,2-anthrachinon,
3-Brom-4-(3,5-di-tert-butyl-4-hydroxyphenyl)-5,5,8,8-tetramethyl-5,6,7,8-tetrahydro-1,2-anthrachinon,
2-(3,5-Di-tert-butyl-4-oxocyclohexa-2,5-dienyliden)-indan-1,3-dion,
2- (3,5-Di-tert-butyl-4-oxocyclohexa-2,5-dienyliden)-3,4-epoxy-3-hydroxy-4-methoxy-3,4-dihydro-2H-naphthalin-1-on,
2-(3,5-Di-tert-butyl-4-oxocyclohexa-2,5-dienyliden)-3,4-epoxy-3,4-dimethoxy-3,4-dihydro-2H-naphthalin-1-on,
2-(3,5-Di-tert-butyl-4-hydroxyphenyl)-indan-1-on,
3,3-Bi-[2-(3,5-di-tert-butyl-4-hydroxyphenyl)-inden-1-on]-3-yl,
2-(3,5-Di-tert-butyl-4-hydroxyphenyl)-3-brom-5,5,8,8-tetramethyl-5,6,7,8-tetrahydro-1,4-anthrachinon,
2-(3,5-Di-tert-butyl-4-hydroxyphenyl)-3-chlor-5,5,8,8-tetramethyl-5,6,7,8-tetrahydro-1,4-anthrachinon,
2-(3,5-Di-tert-butyl-4-hydroxypheayl)-3-methoxy-5,5,8,8-tetramethyl-5,6,7,8-tetrahydro-1,4-anthrachinon,
2-(3,5-Di-tert-butyl-4-hydroxyphenyl)-3-hydroxy-5,5,8,8-tetramethyl-5,6,7,8-tetrahydro-1,4-anthrachinon,
2-(3,5-Di-tert-butyl-4-hydroxyphenyl)-5,5,8,8-tetramethyl-5,6,7,8-tetrahydro-1,4-anthbachinon,
2-Brom-3-(3-brom-5-tert-butyl-4-hydroxyphenyl)-5,5,8,8-tetramethyl-5,6,7,8-tetrahydro-1,4-anthrachinon,
2-Brom-3-(3,5-dibrom-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-3-hydroxy-5,5,8,8-tetramethyl-5,6,7,8-tetrahydro-1,4-anthrachinon.
3-Brom-2-(3,5-di-tert-butyl-4-hydroxyphenyl)-1,4-anthrachinon,
2- (3,5-Di-tert-butyl-4-hydroxyphenyl)-3-methoxy-1,4-anthrachinon,
2-(3,5-Di-tert-butyl-4-hydroxyphenyl)-3-hydroxy-1,4-anthrachinon,
5,5,8,8-Tetramethyl-5,6,7,8-tetrahydronaphthalin-1,3-diol,
3-Methoxy-5,5,8,8-tetramethyl-5,6,7,8-tetrahydronaphthalin-1-ol,
4-(3-Chlor-5,5,8,8-tetramethyl-1,4-dioxo-1,4,5,6,7,5-hexahydroanthracen-2-yl)-benzoesäure,
Methyl-4-(3-chlor-5,5,8,8-tetramethyl-1,4-dioxo-1,4,5,6,7,8-hexahydroanthracen-2-yl)-benzoat, 4-(3-Hydroxy-1,4-dioxo-1,4-dihydronaphthalin-2-yl)-benzoesäure,
Methyl-(3-methoxy-1,4-dioxo-1,4-dihydronaphthalin-2-yl)-benzoesäure,
4-(3-Hydroxy-5,5,8,8-tetramethyl-1,4-dioxo-1,4,5,6,7,8-hexahydroanthracen-2-yl)-benzoesäure, Methyl-4-(3-hydroxy-1,4-dioxo-1,4-dihydronaphthalin-2-yl-azo)-benzoat,
4-(3-Hydroxy-5,5,8,8-tetramethyl-1,4-dioxo-1,4,5,6,7,8-hexahydroanthracen-2-yl-azo)-benzoesäure,
3-(3,5-Di-tert-butyl-4-oxocyclohexa-2,5-dienyliden)-5,5,8,8-tetramethyl-5,6,7,8-tetrahydrocyclopenta [b] naphthalin-1,2-dion,
3-(3,5-Di-tert-butyl-4-oxocyclohexa-2,5-dienyliden)-5,5,8,8-tetramethyl-5,6,7,8-tetrahydroanthracen-3H-1,2,4-trion,
2-(3,5-Di-tert-butyl-4-hydroxyphenyl)-3-methoxy-5,8-dimethyl-1,4-naphthochinon,
2-(3,5-Di-tert-butyl-4-hydroxyphealyl)-3-methoxy-6,7-dimethyl-1,4-naphthochinon,
2-(3,5-Di-tert-butyl-4-hydroxyphenyl)-3-methoxy-5-methyl-1,4-naphthochinon,
2-(3,5-Di-tert-butyl-4-hydroxyphenyl)-2-methoxy-5-methyl-1,4-naphthochinon,
2-(3,5-Di-tert-butyl-4-hydroxyphenyl)-3-methoxy-6-methyl-1,4-naphthochinon,
3-(3,5-Di-tert-butyl-4-hydroxyphenyl)-2-methoxy-6-methyl-1,4-naphthochinon,
2-(3,5-Di-tert-butyl-4-hydroxyphenyl)-3-methoxy-5,6-dimethyl-1,4-naphthochinon,
3-(3,5-Di-tert-butyl-4-hydroxyphenyl)-2-methoxy-5,6-dimethyl-1,4-naphthochinon,
2-(3,5-Di-tert-butyl-4-hydroxyphenyl)-3-methoxy-5,7-dimethyl-1,4-naphthochinon,
3- (3,5-Di-tert-butyl-4-hydroxyphenyl) -2-methoxy-5,7-dimethyl-1,4-naphthochinon,
2-(3,5-Di-tert-butyl-4-hydroxyphenyl)-3-ethylthio-5-methyl-1,4-naphthochinon,
2-(3,5-Di-tert-butyl-4-hydroxyphenyl)-3-ethylthio-6-methyl-1,4-naphthochinon,
2-(3,5-Di-tert-butyl-4-hydxoxyphenyl)-3-hydroxy-5,8-dimethyl-1,4-naphthochinon,
2-(3,5-Di-tert-butyl-4-hydroxyphenyl)-3-hydroxy-6,7-dimethyl-1,4-naphthochinon,
2- (3, 5-Di-tert-butyl-4-hydroxyphenyl)-3-hydroxy-5-methyl-1,4-naphthochinon,
3-(3,5-bi-tert-butyl-4-hydroxyphenyl)-2-hydroxy-5-methyl-1,4-naphthochinon,
2-(3,5-Di-tert-butyl-4-hydroxyphenyl)-3-hydroxy-6-methyl-1,4-naphthochinon,
3-(3,5-Di-tert-butyl-4-hydroxyphenyl)-2-hydroxy-6-methyl-1,4-naphthochinon,
2-(3,5-Di-tert-butyl-4-hydroxyphenyl)-3-hydroxy-5,6-dimethyl-1,4-naphthochinon,
2-(3-Brom-B-tert-butyl-4-hydroxyphenyl)-3-hydroxy-5,6-dimethyl-1l,4-naphthochinon,
3-(3,5-Di-tert-butyl-4-hydroxyphenyl)-2-hydroxy-5,6-dimethyl-1,4-naphthochinon,
2-(3,5-Di-tert-butyl-4-hydroxyphanyl)-3-hydroxy-5,7-dimethyl-1,4-naphthochinon,
3-(3,5-Di-tert-butyl-4-hydroxyphenyl)-2-hydroxy-5,7-dimethyl-1,4-naphthochinon.

4. verfahren nach einem der voranstehenden Ansprüche, **dadurch gekennzeichnet, dass** man die genomische DNA-Probe vor der Behandlung thermisch denaturiert.

5. Verfahren nach Anspruch 1 **dadurch gekennzeichnet, dass** man den Schritt c) in zwei Teilschritten wie folgt durchführt:
a) eine PCR Präamplifikation mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben;
b) eine PCR Amplifikation des in der Präamplifikation gebildeten Produkts mit Primern unterschiedlicher Sequenz, die jeweils zu einem Abschnitt der nach Anspruch 1 vorbehandelten DNA-Probe [(+)-Strang oder (-)-Strang] identisch oder komplementär sind und die zu amplifizierende DNA spezifisch hybridisieren.

6. verfahren nach einem der vorangehenden Ansprüche, **dadurch gekennzeichnet, dass** man die Amplifikation von mehreren DNA-Abschnitten in einem Reaktionsgefäß durchführt.

7. Verfahren nach einem der vorangehenden Ansprüche, **dadurch gekennzeichnet, dass** man für die Polymerasereaktion eine hitzebeständige DNA-Polymerase verwendet.

8. verfahren nach einem der vorangehenden Ansprüche, **dadurch gekennzeichnet, dass** man für die Detektion der vorbehandelten DNA die PCR-Produkte auf einen Oligonukleotid Array hybridisiert und man anschließend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) man das Oligonukleotid mit bekannter Sequenz von n Nukleotiden mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

9. Verfahren nach einem der Ansprüche 1 bis 7 **dadurch gekennzeichnet, dass** man für die Detektion der vorbehandelten DNA die PCR-Produkte auf einen oligonukleotid Array hybridisiert und man anschließend die folgenden Teilschritte ausgeführt:
(a) man hybridisiert einen Satz von oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3' - Ende des hybridisierten oligonukleotids der ersten Spezies an der Stelle der besagten ausgewählten Position getrennt ist;
(b) man das Oligonukleotid der ersten Spezies mit bekannter Sequenz von n Nukleotiden mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man **dadurch** ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

10. Verfahren nach Anspruch 9, **dadurch gekennzeichnet, dass** die verwendeten Oligonukleotide der ersten Spezies und/oder die verwendeten Oligonukleotide der zweiten Spezies entweder nur die Basen T, A und C oder aber die Basen T, A und G enthalten.

11. Verfahren nach einem der Ansprüche 1 bis 7, **dadurch gekennzeichnet, dass** man für die Detektion der vorbehandelten DNA die PCR-Produkte auf einen oligonukleotid Array hybridisiert und man anschließend die folgenden Teilschritte ausführt:
(a) man hybridisiert die amplifizierte genomiache DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) man das Oligonukleotid, sofern es mit seinem 3'-Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

12. Verfahren nach einem der voranstehenden Ansprüche, **dadurch gekennzeichnet, dass** man die PCR-Produkte und/oder Verlängerungsprodukte und/oder Ligationsprodukte für die Detektion mit einer nachweisbaren Markierung versieht.

13. Verfahren nach einem der voranstehenden Ansprüche, **dadurch gekennzeichnet, dass** die Markierungen Fluoreszenzmarkierungen sind.

14. Verfahren nach einem der voranstehenden Ansprüche, **dadurch gekennzeichnet, dass** die Markierungen Radionuklide sind.

15. Verfahren nach einem der Ansprüche 1 bis 12, **dadurch gekennzeichnet, dass** die Markierungen der Nukleotide ablösbare Massenmarkierungen sind, die in einem Massenspektrometer nachweisbar sind.

16. Verfahren nach einem der Ansprüche 1 bis 12, **dadurch, gekennzeichnet, dass** man die PCR-Produkte und/oder Verlängerungsprodukte und/oder Ligationsprodukte insgesamt im Massenspektrometer nachweist und somit durch ihre Masse eindeutig charakterisiert sind.

17. Verfahren nach einem der Ansprüche 1 bis 12, **dadurch gekennzeichnet, dass** man jeweils ein Fragment der PCR-Produkte und/oder Verlängerungsprodukte und/oder Ligationsprodukte im Massenspektrometer nachweist.

18. verfahren nach Anspruch 17, **dadurch gekennzeichnet, dass** man das Fragment des PCR-Produkts und/oder Verlängerungsprodukts und/oder Ligationsprodukts durch Verdau mit einer oder mehrerer Exo- oder Endonukleasen erzeugt.

19. Verfahren nach Anspruch 17 und 18, **dadurch gekennzeichnet, dass** man zur besseren Detektierbarkeit im Massenspektrometer die erzeugten Fragmente mit einer einzelnen positiven oder negativen Nettoladung versieht.

20. Verfahren gemäß einem der voranstehenden Ansprüche, **dadurch gekennzeichnet, dass** man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

21. Verfahren nach einem der voranstehenden Ansprüche, wobei man die genomische DNA aus einer DNA-Probe erhält, wobei Quellen für DNA z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

22. Verwendung eines verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische und psychologische Konsequenzen von Gehirnschädigungen; psychotische Störungen und PersönlichkeitsStörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion ; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

23. Verwendung eines Verfahrens nach einem der voranstehenden Ansprüche zur Unterscheidung von Zelltypen oder Geweben oder zur Untersuchung der Zelldifferenzierung.

24. Kit zur Durchführung eines Assays nach einem der Ansprüche 1 bis 21, bestehend aus einem Bisulfit enthaltenen Reagenz, denaturierenden Lösungsmitteln, sowie Radikalfängern und Primern zur Herstellung der Amplifikate.

## Claims

1. A method for the detection of cytosine methylations in DNA is hereby **characterized in that** the following operating steps are conducted:
a) a genomic DNA sample is incubated with a solution of a bisulfite (= hydrogen sulfite, disulfite) in the concentration range between 0.1 and 6 mol/litre, whereby a denaturing solvent as well as at least one radical scavenger is present;
b) the treated DNA sample is diluted with water or an aqueous solution;
c) the DNA sample is amplified in a polymerase reaction;
d) whereby one desulfonates the DNA before step c;
e) one detects how much the sequence has changed by the treatment according to step a) in comparison to the genomic DNA sample and concludes the methylation state of at least one locus in the genomic DNA sample.

2. The method according to claim 1, further **characterized in that** the denaturing reagent and/or solvent is selected from the following list of compounds or compound classes:
Polyethylene glycol dialkyl ethers, dioxane and substituted derivatives, acetonitrile, primary alcohols, secondary alcohols, tertiary alcohols, diethylene glycol dialkyl ethers, triethylene glycol dialkyl ethers, tetraethylene glycol dialkyl ethers, pentaethylene glycol dialkyl ethers, hexaethylene glycol dialkyl ethers, DMSO, THF.

3. The method according to claim 1 or 2, further **characterized in that** the radical scavenger is selected from the following group of compounds:
Di- and trihyroxybenzenes, green tea extract, pine bark extract, gingko biloba extract, (EGb 761), flavonoid mixture of different fruit and vegetable extracts (GMLD), Bio-Normalizer (Sun-O Corp), DPPH (1,1-diphenyl-2-picrylhydrazyl), NDGA (nondihydroguaiaretic acid), Trolox (6-hydroxy-2,5-7,8-tetramethylchromine 2-carboxylic acid), 2,6-di-tert-butylphenol, 4-methyl-di-tert-butylphenol, 4-methoxy-di-tert-butylphenol, 2,6-di-tert-butyl-p-cresol, 3,4-dihydroxybenzoic acid, vitamin C, vitamin E, vitamin Q, hydroquinone, ubiquinone, lignans, hydroxyterpenes, flavonoids, curcumin, tannins, retinoic acid compounds, Ge-132 bis-betacarboxyethylgermanium sesquioxide, superoxide dismutase (SOD), superoxide catylase, alphanaphthoflavone, di(2-methyl-5-chlorophenyl)dithionate and Cu(II) derivatives, mebendazole, CS (chloroformsoluble) alkyloid extract,
4-(3,5-di-tert-butyl-4-hydroxyphenyl) 3-hydroxy-1,2-naphthoquinone,
4-(3,5-di-tert-butyl-4-hydroxyphenyl) 3-methoxy-1,2-naphthoquinone,
4-(3,5-di-tert-butyl-4-hydroxyphenyl) 1,2-naphthoquinone,
2-(3,5-di-tert-butyl-4-hydroxyphenyl) 3-bromo-1,4-naphthoquinone,
2-(3,5-di-tert-butyl-4-hydroxyphenyl) 3-chloro-1,4-naphthoquinone,
2-(3,5-di-tert-butyl-4-hydroxyphenyl) 3-methoxy-1,4-naphthoquinone,
2-(3,5-di-tert-butyl-4-hydroxyphenyl) 3-hydroxy-1,4-naphthoquinone,
2-(3,5-di-tert-butyl-4-hydroxyphenyl) 1,4-naphthoquinone,
4-(3,5-di-tert-butyl-4-hydroxyphenyl) 3-hydroxy-5,5,8,8-tetramethyl-5,6,7,8-tetrahydro-1,2-anthraquinone,
4-(3,5-di-tert-butyl-4-hydroxyphenyl) 3-methoxy-5,5,8,8-tetramethyl-5,6,7,8-tetrahydro-1,2-anthraquinone,
4-(3,5-di-tert-butyl-4-hydroxyphenol)-5,5,8,8-tetramethyl-5,6,7,8-tetrahydro-1,2-anthraquinone,
3-bromo-4-(3,5-di-tert-butyl-4-hydroxyphenyl)-5,5,8,8-tetramethyl-5,6,7,8-tetrahydro-1,2-anthraquinone,
2-(3,5-di-tert-butyl-4-oxocyclohexa-2,5-dienylidine) indan-1,3-dione,
2-(3,5-di-tert-butyl-4-oxocyclohexa-2,5-dienylidine) 3,4-epoxy-3-hydroxy-4-methoxy-3,4-dihydro-2H-naphthalen-1-one,
2-(3,5-di-tert-butyl-4-oxocyclohexa-2,5-dianylidine) 3,4-epoxy-3,4-dimethoxy-3,4-dihydro-2H-naphthalen-1-one,
2-(3,5-di-tert-butyl-4-hydroxyphenyl) indan-1-one,
3,3-bi-[2-(3,5-di-tert-butyl-4-hydroxyphenyl)indan-1-on]-3-yl,
2-(3,5-di-tert-butyl-4-hydroxyphenyl) 3-bromo-5,5,8,8-tetramethyl-5,6,7,8-tetrahydro-1,4-anthraquinone,
2-(3,5-di-tert-butyl-4-hydroxyphenyl) 3-chloro-5,5,8,8-tetramethyl-5,6,7,8-tetrahydro-1,4-anthraquinone,
2-(3,5-di-tert-butyl-4-hydroxyphenyl) 3-methoxy-5,5,8,8-tetramethyl-5,6,7,8-tetrahydro-1,4-anthraquinone,
2-(3,5-di-tert-butyl-4-hydroxyphenyl) 3-hydroxy-5,5,8,8-tetramethyl-5,6,7,8-tetrahydro-1,4-anthraquinone,
2-(3,5-di-tert-butyl-4-hydroxyphenyl)-5,5,8,8-tetramethyl-5,6,7,8-tetrahydro-1,4-anthraquinone, 2-bromo-3-(3-bromo-5-tert-butyl-4-hydroxyphenyl)-5,5,8,8-tetramethyl-5,6,7,8-tetrahydro-1,4-anthraquinone,
2-bromo-3-(3,5-dibromo-4-hydroxyphenyl)-5,5,8,8-tetramethyl-5,6,7,8-tetrahydro-1,4-anthraquinone,
2-bromo-3-(3-bromo-5-tert-butyl-4-hydroxyphenyl) 3-hydroxy-5,5,8,8-tetramethyl-5,6,7,8-tetrahydro-1,4-anthraquinone,
3-bromo-2-(3,5-di-tert-butyl-4-hydroxyphenyl) 1,4-anthroquinone,
2-(3,5-di-tert-butyl-4-hydroxyphenyl) 3-methoxy-1,4-anthraquinone,
2-(3,5-di-tert-butyl-4-hydroxyphenyl) 3-hydroxy-1,4-anthraquinone,
5,5,8,8-tetramethyl-5,6,7,8-tetrahydronapthalen-1,3-diol,
3-methoxy-5,5,8,8-tetramethyl-5,6,7,8-tetrahydronaphthalen-1-ol,
4-(3-chloro-5,5,8,8-tetramethyl-1,4-dioxo-1,4,5,6,7,8-hexahydroanthracen-2-yl) benzoic acid,
methyl-4-(3-chloro-5,5,8,8-tetramethyl-1,4-dioxo-1,4,5,6,7,8-hexahydroanthracen-2-yl) benzoate,
4-(3-hydroxy-1,4-dioxo-1,4-dihydronaphthalen-2-yl) benzoic acid,
methyl-(3-methoxy-1,4-dioxo-1,4-dihydronaphthalen-2-yl) benzoic acid,
4-(3-hydroxy-5,5,8,8-tetramethyl-1,4-dioxo-1,4,5,6,7,8-hexahydroanthracen-2-yl) benzoic acid,
methyl-4-(3-hydroxy-1,4-dioxo-1,4-dihydronaphthalen-2-yl-azo) benzoate,
4-(3-hydroxy-5,5,8,8-tetramethyl-1,4-dioxo-1,4,5,6,7,8-hexahydroanthracen-2-yl-azo) benzoic acid,
3-(3,5-di-tert-butyl-4-oxocyclohexa-2,5-dienylidine) 5,5,8,8-tetramethyl-5,6,7,8-tetrahydrocyclopenta[b]naphthalne-1,2-dione,
3-(3,5-di-tert-butyl-4-oxocyclohexa-2,5-dienylidene)
5,5,8,8-tetramethyl-5,6,7,8-tetrahydroanthracen-3H-1,2,4-trione,
2-(3,5-di-tert-butyl-4-hydroxyphenyl) 3-methoxy-5,8-dimethyl-1,4-naphthoquinone,
2-(3,5-di-tert-butyl-4-hydroxyphenyl) 3-methoxy-6,7-dimethyl-1,4-naphthoquinone,
2-(3,5-di-tert-butyl-4-hydroxyphenyl) 3-methoxy-5-methyl-1,4-naphthoquinone,
2-(3,5-di-tert-butyl-4-hydroxyphenyl) 2-methoxy-5-methyl-1,4-naphthoquinone,
2-(3,5-di-tert-butyl-4-hydroxyphenyl) 3-methoxy-6-methyl-1,4-naphthoquinone,
3-(3,5-di-tert-butyl-4-hydroxyphenyl) 2-methoxy-6-methyl-1,4-naphthoquinione,
2-(3,5-di-tert-butyl-4-hydroxyphenyl) 3-methoxy-5,6-dimethyl-1,4-naphthoquinione,
3-(3,5-di-tert-butyl-4-hydroxyphenyl) 2-methoxy-5,6-dimethyl-1,4-naphthoquinone,
2-(3,5-di-tert-butyl-4-hydroxyphenol)-3-methoxy-5,7-dimethyl-1,4-naphthoquinone,
3-(3,5-di-tert-butyl-4-hydroxyphenyl) 2-methoxy-5,7-dimethyl-1,4-naphthoquinone,
2-(3,5-di-tert-butyl-4-hydroxyphenyl) 3-ethylthio-5-methyl-1,4-naphthoquinone,
2-(3,5-di-tert-butyl-4-hydroxyphenyl) 3-ethylthio-6-methyl-1,4-naphthoquinone,
2-(3,5-di-tert-butyl-4-hydroxyphenyl)-3-hydroxy-5,8-dimethyl-1,4-naphthoquinone,
2-(3,5-di-tert-butyl-4-hydroxyphenyl)-3-hydroxy-6,7-dimethyl-1,4-naphthoquinone,
2-(3,5-di-tert-butyl-4-hydroxyphenyl) 3-hydroxy-5-methyl-1,4-naphthoquinone,
3-(3,5-di-tert-butyl-4-hydroxyphenyl)-2-hydroxy-5-methyl-1,4-naphthoquinone,
2-(3,5-di-tert-butyl-4-hydroxyphenyl)-3-hydroxy-6-methyl-1,4-naphthoquinone,
3-(3,5-di-tert-butyl-4-hydroxyphenyl)-2-hydroxy-6-methyl-1,4-naphthoquinone,
2-(3,5-di-tert-butyl-4-hydroxyphenyl) 3-hydroxy-5,6-dimethyl-1,4-naphthoquinone,
2-(3-bromo-5-tert-butyl-4-hydroxyphenyl) 3-hydroxy-5,6-dimethyl-1,4-naphthoquinone,
3-(3,5-di-tert-butyl-4-hydroxyphenyl)-2-hydroxy-5,6-dimethyl-1,4-naphthoquinone,
2-(3,5-di-tert-butyl-4-hydroxyphenyl) 3-hydroxy-5,7-dimethyl-1,4-naphthoquinone,
3-(3,5-di-tert-butyl-4-hydroxyphenyl) 2-hydroxy-5,7-dimethyl-1,4-naphthoquinone.

4. The method according to one of the preceding claims, further **characterized in that** the genomic DNA sample is thermally denatured prior to the treatment.

5. The method according to claim 1, further **characterized in that** step c) is conducted in two substeps as follows:
a) a PCR preamplification with at least one pair of primers of different sequence, which hybridize nonspecifically to a DNA sample pretreated according to claim 1 and thus more than one amplified product is produced in the PCR step;
b) a PCR amplification of the product formed in the preamplification with primers of different sequence, which are identical each time to a segment of the DNA sample [(+)-strand or (-)-strand], which has been pretreated according to claim 1 or are complementary [to it], and which specifically hybridize to the DNA to be amplified.

6. The method according to one of the preceding claims, further **characterized in that** the amplification of several DNA segments is conducted in one reaction vessel.

7. The method according to one of the preceding claims, further **characterized in that** a heat-stable DNA polymerase is used for the polymerase reaction.

8. The method according to one of the preceding claims, further **characterized in that** the PCR products are hybridized to an oligonucleotide array for the detection of the pretreated DNA and then the following sub-steps are conducted:
a) the amplified genomic DNA is hybridized to at least one oligonucleotide with the formation of a duplex, whereby said hybridized oligonucleotides are directly adjacent by their 3' end or at a distance of up to 10 bases to the positions that are to be investigated relative to their methylation in the genomic DNA sample;
(b) the oligonucleotide with known sequence of n nucleotides is extended by at least one nucleotide by means of a polymerase, whereby the nucleotide bears a detectable label and the extension depends on the methylation state of the respective cytosine in the genomic DNA sample.

9. The method according to one of claims 1 to 7, further **characterized in that** the PCR products are hybridized to an oligonucleotide array for the detection of the pretreated DNA and then the following sub-steps are conducted:
(a) a set of oligonucleotides is hybridized to the amplified genomic DNA with the formation of a duplex, whereby this set of oligonucleotides is comprised of two different species and whereby the hybridized oligonucleotides of the first species are directly adjacent by their 3' end or at a distance of up to 10 bases to the positions that are to be investigated relative to their methylation in the genomic DNA sample, and whereby the second oligonucleotide of the second species hybridizes to a second region of the target molecule, so that the 5' end of the oligonucleotide of the second species is separated by a gap of the size of one single nucleotide or up to 10 nucleotides from the 3' end of the hybridized oligonucleotide of the first species at the site of said selected position;
(b) the oligonucleotide of the first species with known sequence of n nucleotides is extended by means of a polymerase by at most the number of nucleotides that lie between the 3' end of the oligonucleotide of the first species and the 5' end of the oligonucleotide of the second species, wherein the extension depends on the methylation state of the respective cytosine in the genomic DNA sample;
(c) the oligonucleotides are incubated in the presence of a ligase, whereby the adjacent oligonucleotide of the first species that has been extended by the polymerase reaction and the oligonucleotide of the second species are joined and in this way a ligation product is obtained, as long as an elongation of the oligonucleotide of the first species has been produced in the preceding step so that now the 3' end with a present 3' hydroxy function of the extended oligonucleotide is directly adjacent to the 5' end of the oligonucleotide of the second species.

10. The method according to claim 9, further **characterized in that** the oligonucleotides of the first species that are used and/or the oligonucleotides of the second species that are used contain either only the bases T, A and C or the bases T, A and G.

11. The method according to one of claims 1 to 7, further **characterized in that** the PCR products are hybridized to an oligonucleotide array for the detection of the pretreated DNA and then the following sub-steps are conducted:
(a) the amplified genomic DNA is hybridized to at least one oligonucleotide with known sequence of n nucleotides with the formation of a duplex, whereby said hybridized oligonucleotides hybridize by the 3' end either partially or completely to the positions that are to be investigated relative to their methylation in the genomic DNA sample;
(b) as long as the oligonucleotide has hybridized by its 3' terminus beforehand without erroneous base pairing to the position to be investigated, it is extended by at least one nucleotide by means of a polymerase, whereby at least one nucleotide bears a detectable label and the extension depends on the methylation state of the respective cytosine in the genomic DNA sample.

12. The method according to one of the preceding claims, further **characterized in that** the PCR products and/or extension products and/or ligation products are provided with a detectable label for detection.

13. The method according to one of the preceding claims, further **characterized in that** the labels are fluorescent labels.

14. The method according to one of the preceding claims, further **characterized in that** the labels are radionuclides.

15. The method according to one of claims 1 to 12, further **characterized in that** the labels of the nucleotides are removable mass labels, which can be detected in a mass spectrometer.

16. The method according to one of claims 1 to 12, further **characterized in that** the PCR products and/or extension products and/or ligation products are detected as a whole in the mass spectrometer and thus are clearly **characterized by** their mass.

17. The method according to one of claims 1 to 12, further **characterized in that** a fragment of the PCR products and/or extension products and/or ligation products is detected each time in the mass spectrometer.

18. The method according to claim 17, further **characterized in that** the fragment of the PCR product and/or extension product and/or ligation product is produced by digestion with one or more exo- or endonucleases.

19. The method according to claims 17 and 18, further **characterized in that** the produced fragments are provided with a single positive or negative net charge for better detectability in the mass spectrometer.

20. The method according to one of the preceding claims, further **characterized in that** the PCR products and/or extension products and/or ligation products are detected and visualized by means of matrix assisted laser desorption/ionization mass spectrometry (MALDI-TOF) or by means of electrospray mass spectrometry (ESI).

21. The method according to one of the preceding claims, whereby the genomic DNA is obtained from a DNA sample, whereby sources for DNA comprise, e.g., cell lines, blood, sputum, stool, urine, cerebrospinal fluid, tissue embedded in paraffin, for example, tissue of eyes, intestine, kidneys, brain, heart, prostate, lungs, breast or liver, histological slide preparations and all possible combinations thereof.

22. Use of a method according to one of the preceding claims for the diagnosis and/or prognosis of adverse events for patients or individuals, whereby these adverse events belong to at least one of the following categories: undesired drug interactions; cancer diseases; CNS malfunctions, damage or disease; symptoms of aggression or behavioral disturbances; clinical and psychological consequences of brain damage; psychotic disturbances and personality disorders; dementia and/or associated syndromes; cardiovascular disease, malfunction and damage; malfunction, damage or disease of the gastrointestinal tract; malfunction, damage or disease of the respiratory system; lesion, inflammation, infection; malfunction, damage or disease of the body as an abnormality in the development process; malfunction, damage or disease of the skin, of the muscles, of the connective tissue or of the bones; endocrine and metabolic malfunction, damage or disease; headaches or sexual malfunction.

23. Use of a method according to one of the preceding claims for distinguishing cell types or tissues or for investigating cell differentiation.

24. A kit for conducting an assay according to one of the claims 1 to 21, comprising of a reagent containing bisulfite, denaturing solvents, as well as radical scavengers and primers for the production of the amplified products.

## Revendications

1. Procédé pour détecter des méthylations de l'ADN sur la cytosine, **caractérisé en ce qu'**on procède aux étapes suivantes:
a) on incube une sonde ADN génomique avec une solution d'un bisulfite (= hydrogénosulfite, disulfite) dans la plage de concentrations allant de 0, 1 à 6 mol/l, en présence d'un solvant dénaturant, et d'au moins un fixateur de radicaux ;
b) on dilue avec de l'eau ou avec une solution aqueuse la sonde ADN ainsi traitée;
c) on amplifie la sonde ADN dans le cadre d'une réaction de polymérisation ;
d) ce à l'occasion de quoi, avant l'étape c), on procède à une désulfonation de l'ADN ;
e) on détecte dans quelle mesure la séquence s'est modifiée sous l'effet du traitement selon l'étape a) par rapport à la sonde ADN génomique, et on en déduit l'état de méthylation d'au moins un locus de la sonde ADN génomique.

2. Procédé selon la revendication 1, **caractérisé en ce que** le réactif et/ou le solvant dénaturant est choisi dans la liste suivante de composés ou de classes de composés :
éthers dialkyliques du polyéthylèneglycol, dioxanne et dérivés substitués, acétonitrile, alcools primaires, alcools secondaires, alcools tertiaires, éthers dialkyliques du diéthylèneglycol, éthers dialkyliques du triéthylèneglycol, éthers dialkyliques du tétraéthylèneglycol, éthers dialkyliques du pentaéthylèneglycol, éthers dialkyliques de l'hexaéthylèneglycol, DMSO, THF.

3. Procédé selon la revendication 1 ou 2, **caractérisé en ce que** le fixateur de radicaux est choisi dans le groupe de composés suivant :
di-, trihydroxybenzènes, extrait de thé vert (green tea extract), pycnogénol (extrait d'écorce de pin), extrait de Ginkgo Biloba (EGb 761), mélange de flavonoïdes de différents extraits de fruits et de légumes (GNLD), bionormalisateur (Sun-O Corp), DPPH (1,1-diphényl-2-picrylhydrazyle), NDGA (acide nordihydroguaiarétique), trolox (acide 6-hydroxy 2,5,7,8-tétraméthylchromanne-2-carboxylique), 2,6-di-tert-butylphénol, 4-méthyl-di-tert-butylphénol, 4-méthoxy-di-tert-butylphénol, 2,6-di-tert-butyl-p-crésol, acide 3,4-dihydroxybenzoïque, vitamine C, vitamine E, vitamine Q, hydroquinone, ubiquinone, lignanes, hydroxyterpènes, flavonoïdes, curcumin, tannins, dérivés de l'acide rétinoïque, Ge-132 sesquioxyde de bisbêtacarboxyéthyl-germanium, superoxyde-dismutase (SOD), superoxyde-catalase, alpha-naphtoflavone, dithionate de di(2-méthyl-5-chlorophényle) et dérivés du Cu(II), mébendazole, extraits d'alcaloïdes CS (solubles dans le chloroforme),
4-(3,5-di-tert-butyl-4-hydroxyphényl)-3-hydroxy-1,2-naphtoquinone,
4-(3,5-di-tert-butyl-4-hydroxyphényl)-3-méthoxy-1,2-naphtoquinone,
4-(3,5-di-tert-butyl-4-hydroxyphényl)-1,2-naphtoquinone,
2-(3,5-di-tert-butyl-4-hydroxyphényl)-3-bromo-1,4-naphtoquinone,
2-(3,5-di-tert-butyl-4-hydroxyphényl)-3-chloro-1,4-naphtoquinone,
2-(3,5-di-tert-butyl-4-hydroxyphényl)-3-méthoxy-1,4-naphtoquinone,
2-(3,5-di-tert-butyl-4-hydroxyphényl)-3-hydroxy-1,4-naphtoquinone,
2-(3,5-di-tert-butyl-4-hydroxyphényl)-1,4-naphtoquinone,
4-(3,5-di-tert-butyl-4-hydroxyphényl)-3-hydroxy-5,5,8,8-tétraméthyl-5,6,7,8-tétrahydro-1,2-anthraquinone,
4-(3,5-di-tert-butyl-4-hydroxyphényl)-3-méthoxy-5,5,8,8-tétraméthyl-5,6,7,8-tétrahydro-1,2-anthraquinone,
4-(3,5-di-tert-butyl-4-hydroxyphényl)-5,5,8,8-tétraméthyl-5,6,7,8-tétrahydro-1,2-anthraquinone,
3-bromo-4-(3,5-di-tert-butyl-4-hydroxyphényl)-5,5,8,8-tétraméthyl-5,6,7,8-tétrahydro-1,2-anthraquinone,
2-(3,5-di-tert-butyl-4-oxocyclohexa-2,5-diénylidène)-indane-1,3-dione,
2-(3,5-di-tert-butyl-4-oxocyclohexa-2,5-diénylidène)-3,4-époxy-3-hydroxy-4-méthoxy-3,4-dihydro-2H-naphtalène-1-one,
2-(3,5-di-tert-butyl-4-oxocyclohexa-2,5-diénylidène)-3,4-époxy-3,4-diméthoxy-3,4-dihydro-2H-naphtalène-1-one,
2-(3,5-di-tert-butyl-4-hydroxyphényl)-indane-1-one,
3,3-bi-[2-(3,5-di-tert-butyl-4-hydroxyphényl)-indène-1-one]-3-yle,
2-(3,5-di-tert-butyl-4-hydroxyphényl)-3-bromo-5,5,8,8-tétraméthyl-5,6,7,8-tétrahydro-1,4-anthraquinone,
2-(3,5-di-tert-butyl-4-hydroxyphényl)-3-chloro-5,5,8,8-tétraméthyl-5,6,7,8-tétrahydro-1,4-anthraquinone,
2-(3,5-di-tert-butyl-4-hydroxyphényl)-3-méthoxy-5,5,8,8-tétraméthyl-5,6,7,8-tétrahydro-1,4-anthraquinone,
2-(3,5-di-tert-butyl-4-hydroxyphényl)-3-hydroxy-5,5,8,8-tétraméthyl-5,6,7,8-tétrahydro-1,4-anthraquinone,
2-(3,5-di-tert-butyl-4-hydroxyphényl)-5,5,8,8-tétraméthyl-5,6,7,8-tétrahydro-1,4-anthraquinone,
2-bromo-3-(3-bromo-5-tert-butyl-4-hydroxyphényl)-5,5,8,8-tétraméthyl-5,6,7,8-tétrahydro-1,4-anthraquinone,
2-bromo-3-(3,5-dibromo-4-hydroxyphényl-5,5,8,8-tétraméthyl-5,6,7,8-tétrahydro-1,4-anthraquinone,
2-bromo-3-(3-bromo-5-tert-butyl-4-hydroxyphényl)-3-hydroxy-5,5,8,8-tétraméthyl-5,6,7,8-tétrahydro-1,4-anthraquinone,
3-bromo-2-(3,5-di-tert-butyl-4-hydroxyphényl)-1,4-anthraquinone,
2-(3,5-di-tert-butyl-4-hydroxyphényl)-3-méthoxy-1,4-anthraquinone,
2-(3,5-di-tert-butyl-4-hydroxyphényl)-3-hydroxy-1,4-anthraquinone,
5,5,8,8-tétraméthyl-5,6,7,8-tétrahydro-naphtalène-1,3-diol,
3-méthoxy-5,5,8,8-tétraméthyl-5,6,7,8-tétrahydronaphtalène-1-ol,
acide 4-(3-chloro-5,5,8,8-tétraméthyl-1,4-dioxo-1,4,5,6,7,8-hexahydroanthracène-2-yl)-benzoïque,
4-(3-chloro-5,5,8,8-tétraméthyl-1,4-dioxo-1,4,5,6,7,8-hexahydroanthracène-2-yl)-benzoate de méthyle,
acide 4-(3-hydroxy-1,4-dioxo-1,4-dihydro-naphtalène-2-yl)-benzoïque,
acide-(3-méthoxy-1,4-dioxo-1,4-dihydronaphtalène-2-yl)-benzoate de méthyle,
acide 4-(3-hydroxy-5,5,8,8-tétraméthyl-1,4-dioxo-1,4,5,6,7,8-hexahydroanthracène-2-yl)-benzoïque,
4-(3-hydroxy-1,4-dioxo-1,4-dihydronaphtalène-2-yl-azo)-benzoate de méthyle,
acide 4-(3-hydroxy-5,5,8,8-tétraméthyl-1,4-dioxo-1,4,5,6,7,8-hexahydroanthracène-2-yl-azo)-benzoique,
3-(3,5-di-tert-butyl-4-oxocyclohexa-2,5-diénylidène)-5,5,8,8-tétraméthyl-5,6,7,8-tétrahydro-cyclopenta[b]naphtalène-1,2-dione,
3-(3,5-di-tert-butyl-4-oxocyclohexa-2,5-diénylidène)-5,5,8,8-tétraméthyl-5,6,7,8-tétrahydro-anthracène-3H-1,2,4-trione,
2-(3,5-di-tert-butyl-4-hydroxyphényl)-3-méthoxy-5,8-diméthyl-1,4-naphtoquinone,
2-(3,5-di-tert-butyl-4-hydroxyphényl)-3-méthoxy-6,7-diméthyl-1,4-naphtoquinone,
2-(3,5-di-tert-butyl-4-hydroxyphényl)-3-méthoxy-5-méthyl-1,4-naphtoquinone,
2-(3,5-di-tert-butyl-4-hydroxyphényl)-2-méthoxy-5-méthyl-1,4-naphtoquinone,
2-(3,5-di-tert-butyl-4-hydroxyphényl)-3-méthoxy-6-méthyl-1,4-naphtoquinone,
3-(3,5-di-tert-butyl-4-hydroxyphényl)-2-méthoxy-6-méthyl-1,4-naphtoquinone,
2-(3,5-di-tert-butyl-4-hydroxyphényl)-3-méthoxy-5,6-diméthyl-1,4-naphtoquinone,
3-(3,5-di-tert-butyl-4-hydroxyphényl)-2-méthoxy-5,6-diméthyl-1,4-naphtoquinone,
2-(3,5-di-tert-butyl-4-hydroxyphényl)-3-méthoxy-5,7-diméthyl-1,4-naphtoquinone,
3-(3,5-di-tert-butyl-4-hydroxyphényl)-2-méthoxy-5,7-diméthyl-1,4-naphtoquinone,
2-(3,5-di-tert-butyl-4-hydroxyphényl)-3-éthylthio-5-méthyl-1,4-naphtoquinone,
2-(3,5-di-tert-butyl-4-hydroxyphényl)-3-éthylthio-6-méthyl-1,4-naphtoquinone,
2-(3,5-di-tert-butyl-4-hydroxyphényl)-3-hydroxy-5,8-diméthyl-1,4-naphtoquinone,
2-(3,5-di-tert-butyl-4-hydroxyphényl)-3-hydroxy-6,7-diméthyl-1,4-naphtoquinone,
2-(3,5-di-tert-butyl-4-hydroxyphényl)-3-hydroxy-5-méthyl-1,4-naphtoquinone,
3-(3,5-di-tert-butyl-4-hydroxyphényl)-2-hydroxy-5-méthyl-1,4-naphtoquinone,
2-(3,5-di-tert-butyl-4-hydroxyphényl)-3-hydroxy-6-méthyl-1,4-naphtoquinone,
3-(3,5-di-tert-butyl-4-hydroxyphényl)-2-hydroxy-6-méthyl-1,4-naphtoquinone,
2-(3,5-di-tert-butyl-4-hydroxyphényl)-3-hydroxy-5,6-diméthyl-1,4-naphtoquinone,
2-(3-bromo-5-tert-butyl-4-hydroxyphényl)-3-hydroxy-5,6-diméthyl-1,4-naphtoquinone,
3-(3,5-di-tert-butyl-4-hydroxyphényl)-2-hydroxy-5,6-diméthyl-1,4-naphtoquinone,
2-(3,5-di-tert-butyl-4-hydroxyphényl)-3-hydroxy-5,7-diméthyl-1,4-naphtoquinone,
3-(3,5-di-tert-butyl-4-hydroxyphényl)-2-hydroxy-5,7-diméthyl-1,4-naphtoquinone.

4. Procédé selon l'une des revendications précédentes, **caractérisé en ce qu'**on soumet la sonde ADN génomique à une dénaturation thermique avant le traitement.

5. Procédé selon la revendication 1, **caractérisé en ce qu'**on met en oeuvre comme suit l'étape c) en deux étapes partielles :
a) une préamplification PCR avec au moins une paire d'amorces ayant des séquences différentes, qui vont s'hybrider d'une manière non spécifique à une sonde ADN prétraitée selon la revendication 1, et qui donc, dans l'étape PCR, vont donner plus d'un produit d'amplification ;
b) une amplification PCR du produit formé lors de la préamplification, avec des amorces ayant des séquences différentes, dont chacune est identique ou complémentaire d'un segment de la sonde ADN [brin (+) ou brin (-)] prétraitée selon la revendication 1, et qui s'hybrident d'une manière spécifique à l'ADN devant être amplifié.

6. Procédé selon l'une des revendications précédentes, **caractérisé en ce qu'**on met en oeuvre l'amplification de plusieurs segments d'ADN dans un réacteur.

7. Procédé selon l'une des revendications précédentes, **caractérisé en ce qu'**on utilise pour la réaction par polymérase une ADN-polymérase thermorésistante.

8. Procédé selon l'une des revendications précédentes, **caractérisé en ce que**, pour la détection de l'ADN prétraité, on hybride les produits de la PCR sur un arrangement d'oligonucléotides, puis on met en oeuvre les étapes partielles suivantes :
a) l'ADN génomique amplifié est hybridé à au moins un oligonucléotide, avec formation d'un duplex, lesdits oligonucléotides hybridés délimitant, par leur extrémité 3', directement ou avec un écart allant jusqu'à 10 bases, les positions qui doivent faire l'objet d'une étude de leur méthylation dans la sonde ADN génomique ;
b) on prolonge d'au moins un nucléotide l'oligonucléotide ayant la séquence connue de n nucléotides, à l'aide d'une polymérase, le nucléotide portant un marqueur détectable, et le prolongement dépendant de l'état de méthylation de chaque cytosine dans la sonde ADN génomique.

9. Procédé selon l'une des revendications 1 à 7, **caractérisé en ce que**, pour la détection de l'ADN prétraité, on hybride les produits de la PCR à un arrangement d'oligonucléotides, puis on met en oeuvre les étapes partielles suivantes :
(a) on hybride un jeu d'oligonucléotides à l'ADN génomique amplifié avec formation d'un duplex, ce jeu d'oligonucléotides étant constitué de deux espèces différentes, les oligonucléotides hybridés de la première espèce délimitant, par leur extrémité 3', directement ou avec un écart allant jusqu'à 10 bases, les positions qui doivent faire l'objet d'une étude de leur méthylation à la sonde ADN génomique, et le deuxième nucléotide de la deuxième espace s'hybridant à une deuxième région de la molécule cible, de telle sorte que l'extrémité 5' de l'oligonucléotide de la deuxième espèce soit séparée, par un écart ayant la taille d'un nucléotide unique, ou d'un ensemble allant jusqu'à 10 nucléotides, de l'extrémité 3' de l'oligonucléotide hybridé de la première espèce, en le point correspondant à la position sélectionnée mentionnée ;
(b) on prolonge l'oligonucléotide de la première espèce, ayant une séquence connue de n nucléotides, et à l'aide d'une polymérase, au maximum du nombre de nucléotides qui se trouvent entre l'extrémité 3' de l'oligonucléotide de la première espèce et l'extrémité 5' de l'oligonucléotide de la deuxième espèce, le prolongement dépendant de l'état de la méthylation de chaque cytosine de la sonde ADN génomique ;
(c) on incube les oligonucléotides en présence d'une ligase, ce à l'occasion de quoi l'oligonucléotide de la première espèce, adjacent et prolongé par la réaction par polymérase, est assemblé à l'oligonucléotide de la deuxième espèce, et on obtient de ce fait un produit de ligature, du moment que, dans l'étape précédente, un prolongement de l'oligonucléotide de la première espèce a eu lieu de telle sorte que maintenant l'extrémité 3', ayant une fonction 3'-hydroxy de l'oligonucléotide prolongé, soit directement adjacente à l'extrémité 5' de l'oligonucléotide de la deuxième espèce.

10. Procédé selon la revendication 9, **caractérisé en ce que** les oligonucléotides utilisés de la première espèce et/ou les oligonucléotides utilisés de la deuxième espèce ou bien ne contiennent que les bases T, A et C, ou bien contiennent cependant les bases T, A et G.

11. Procédé selon l'une des revendications 1 à 7, **caractérisé en ce que**, pour la détection de l'ADN prétraité, on hybride les produits de la PCR sur un arrangement d'oligonucléotides, puis on met en oeuvre les étapes partielles suivantes :
(a) on hybride l'ADN génomique amplifié à au moins un oligonucléotide ayant une séquence connue de n nucléotides, avec formation d'un duplex, lesdits oligonucléotides hybridés s'hybridant en totalité ou en partie, par leur extrémité 3', aux positions dont il s'agit d'étudier la méthylation dans la sonde ADN génomique ;
(b) à l'aide d'une polymérase, on prolonge l'oligonucléotide d'au moins un nucléotide, du moment que, par son site 3'-terminal, il a été au préalable, sans mésappariements de bases, hybridé à la position devant être étudiée, ce à l'occasion de quoi au moins un nucléotide porte un marqueur détectable, et le prolongement dépend de l'état de la méthylation de chaque cytosine de la sonde ADN génomique.

12. Procédé selon l'une des revendications précédentes, **caractérisé en ce que**, pour la détection, on pourvoit d'un marqueur détectable les produits de la PCR et/ou les produits du prolongement et/ou les produits de la ligature.

13. Procédé selon l'une des revendications précédentes, **caractérisé en ce que** les marqueurs sont des marqueurs fluorescents.

14. Procédé selon l'une des revendications précédentes, **caractérisé en ce que** les marqueurs sont des radionucléides.

15. Procédé selon l'une des revendications 1 à 12, **caractérisé en ce que** les marqueurs des nucléotides sont des marqueurs de masse détachables, qui peuvent être détectés dans un spectromètre de masse.

16. Procédé selon l'une des revendications 1 à 12, **caractérisé en ce qu'**on détecte dans leur totalité, au spectromètre de masse, les produits de la PCR et/ou les produits du prolongement et/ou les produits de la ligature, et ainsi on les caractérise d'une manière non ambiguë par leur masse.

17. Procédé selon l'une des revendications 1 à 12, **caractérisé en ce qu'**on détecte dans chaque cas, au spectromètre de masse, un fragment des produits de la PCR et/ou des produits du prolongement et/ou des produits de la ligature.

18. Procédé selon la revendication 17, **caractérisé en ce qu'**on produit le fragment du produit de la PCR et/ou du produit du prolongement et/ou du produit de la ligature par digestion avec une ou plusieurs exo- ou endonucléases.

19. Procédé selon les revendications 17 et 18, **caractérisé en ce que**, pour améliorer la possibilité de détection au spectrophotomètre, on pourvoit les fragments produits d'une charge nette individuelle, positive ou négative.

20. Procédé selon l'une des revendications précédentes, **caractérisé en ce qu'**on détecte et on visualise les produits de la PCR et/ou les produits du prolongement et/ou les produits de la ligature, par une spectrométrie de masse à désorption/ionisation laser assistée par une matrice (MALDI-TOF), ou par une spectrométrie de masse par électropulvérisation (ESI).

21. Procédé selon l'une des revendications précédentes, dans lequel on obtient l'ADN génomique à partir d'une sonde ADN, les sources de l'ADN englobant par exemple les lignées cellulaires, le sang, les crachats, les selles, l'urine, le liquide céphalo-rachidien, un tissu enrobé de paraffine, par exemple le tissu des yeux, de l'intestin, des reins, du cerveau, du coeur, de la prostate, des poumons, du sein ou du foie, des coupes histologiques, et toutes les combinaisons possibles de ceux-ci.

22. Utilisation d'un procédé selon l'une des revendications précédentes pour le diagnostic et/ou le pronostic d'événements défavorables pour des patients ou des individus, ces événements indésirables appartenant au moins à l'une des catégories suivantes : les effets secondaires indésirables des médicaments ; les maladies de type cancer ; les dysfonctionnements, lésions ou maladies du SNC ; les symptômes d'agression ou les troubles du comportement ; les conséquences cliniques et psychologiques de lésions cérébrales ; les troubles psychotiques et les troubles de la personnalité ; la démence et/ou les syndromes associés ; les maladies, dysfonctionnements et lésions du système cardiovasculaire ; les dysfonctionnements, lésions ou maladies du tractus gastro-intestinal ; les dysfonctionnements, lésions ou maladies du système respiratoire ; les blessures, les inflammations, les infections ; les dysfonctionnements, lésions ou maladies de l'organisme représentant des aberrations du processus de développement ; les dysfonctionnements, lésions ou maladies de la peau, des muscles, du tissu conjonctif ou des os ; les dysfonctionnements, lésions ou maladies endocriniennes et métaboliques ; les céphalées ou les dysfonctionnements sexuels.

23. Utilisation d'un procédé selon l'une des revendications précédentes pour différencier des types cellulaires ou des tissus, ou pour étudier la différenciation cellulaire.

24. Kit destiné à la mise en oeuvre d'une analyse selon l'une des revendications 1 à 21, consistant en un réactif contenant du bisulfite, des solvants dénaturants, ainsi que des fixateurs de radicaux et des amorces, destinés à la préparation des produits d'amplification.
